# EUROPEAN PATENT APPLICATION

(11) **EP 2 387 984 A1**
(43) Date of publication of application: **23.11.2011**
(21) Application number: 10163629.8
(22) Date of filing: 21.05.2010
(51) Int. Cl.: A61K 6/027

(54) **Dental ceramic article, process for production and use thereof**

(71) Applicant: 3M Innovative Properties Company, Saint Paul, MN 55133-3427 (US)
(72) Inventor: The designation of the inventor has not yet been filed
(74) Representative: Brem, Roland

(57) **Abstract**

The invention relates to a dental article comprising two sections, section A and section B, section A having an upper and a lower surface portion, section A comprising zirconia in an amount of at least about 80 mol%, a stabilizer and a component X comprising Dy or Sm or Eu or mixtures thereof, section B comprising a glass, glass ceramic or composite material, section B being located above the upper surface portion of section A.

The invention also relates to a process for producing such an article, a kit of parts and the use of blanks for producing such an article.

## Description

### Field of the Invention

The invention generally relates to a ceramic dental article. The dental article can be used e.g. in the field of dentistry for producing dental crowns and bridges.

### Background Art

Dental restorations containing oxide ceramics are known since a couple of years. Especially zirconia has been used for manufacturing dental ceramic support structures. The shaping of the dental ceramic is typically done using CAD/CAM technology. For the production of aesthetic dental restorations based on oxide ceramics different processes have been suggested.

US 6,709,694 relates to the coloring of ceramics by means of ionic or complex-containing solutions.

US 6,713,421 relates to blanks comprising zirconium oxide-based ceramic with an addition of 0.1 to 0.50 wt% of at least one of the oxides of the elements aluminium, gallium, germanium, indium and their use.

US 2007/0292597 relates to compositions based on ZrO₂ and single- and multi-colored blanks made from oxide ceramics and a process for their preparation, in which oxide ceramic powder is coated with a colouring substance.

EP 1 818 318 A2 describes a production method of a dental ceramics material comprising the step of mixing a pink coloring agent and a yellow coloring agent at a certain mixing ratio with zirconium oxide. As the pink coloring agent manganese oxide is used and as the yellow coloring agent vanadium oxide is used.

US 5,263,858 is directed to an ivory-colored zirconia sintered body containing stabilizer-containing ZrO₂ as a main component and also containing certain amounts of Er₂O₃, Pr₆O₁₁, Fe₂O₃ and ZnO.

There is still room for improvement especially with regard to the requirements to be fulfilled with respect to modern dental materials. Many of the commercially available dental restorations still do not show the appearance of natural dental teeth.

Thus, it is generally an object of the invention to provide a dental article which can be used for making aesthetic dental restorations.

### Description of the Invention

Dental restorations typically comprise two main sections: a support structure (e.g. made out of metal or a ceramic) and a veneering or facing. When producing dental restorations nowadays the dental technician faces the following dilemma. On the one hand a highly translucent veneering or facing is desired to match the natural appearance of the tooth. On the other hand the brightness (sometime also referred to as "value" or "grey value", or "L* value") of the tooth colour should exactly be adapted to the brightness of the neighbouring teeth.

The brightness of the dental restoration can e.g. be increased by decreasing the translucency (i.e. increasing of opacity) of the veneering material. A less translucent material typically scatters and reflects more light.

However, dental restorations then may have the desired brightness but their aesthetic appearance suffers from a too high opacity of the veneering material.

With materials available today (e.g. those where the dental support structure is made of zirconia and where the veneering material is made of pigmented glass or a glass ceramic) it is very difficult, to reach the desired level of translucency and brightness simultaneously.

The present invention addresses this topic.

The dental article described in the present text comprises a fluorescent zirconia containing material.

In one embodiment the invention features a dental article, the dental article comprising two sections, section A and section B,
section A having an upper and a lower surface portion, section A comprising
   zirconia in an amount of at least about 80 mol%,
   a stabilizer and
   a component X comprising Dy or Sm or Eu or mixtures thereof,
section B comprising a glass, glass ceramic or composite material, section B being located above the outer surface portion of section A.

In another embodiment, the invention relates to a process of producing the dental article as described in the present text.

A further aspect is directed to a kit of parts comprising part 1 and part 2,
part 1 comprising a blank comprising zirconia in an amount of at least about 80 mol%, a stabilizer and a component X being selected from Dy or Sm or Eu or mixtures thereof,
part 2 comprising a glass, glass ceramic or composite material,
wherein the blank is to be used for the production of a dental article as described in the present text.

Moreover, the invention features the use of a blank for producing a dental article as described in the present text.

Unless otherwise specified, within the description of the present invention, the following terms have the following meaning:
The term "dental article" is to be understood as an article which can and is to be used in the dental and/or orthodontic area including dental laboratories. In view of regulatory requirements dental articles have to fulfil certain standards. A typical example of a dental article is a dental restoration.
The term "dental restoration" means any restoration which can be used in the dental field. In this respect, the dental restoration shall have sufficient strength. Dental restorations are typically comprised of at least two sections: a dental support structure and a dental veneer, or facing or a coating e.g. with a dental composite material. Examples include crowns, abutments and bridges (including 2, 3, 4, 5, 6, 7 or even 8 parts bridges).

Dental support structures are typically made of or comprise oxide ceramic materials including ZrO₂ or Al₂O₃. Compared to other framework such as pottery or paving stones, the dental support structure is small and filigree and of high strength. The thickness of the dental support structure can vary from very thin, e.g. at the edges and rims (below about 0.1 mm) to considerably thick, e.g. in the biting area (up to about 7 mm). Dental support structures do typically not comprise a glass ceramic material.

"Glass" means an inorganic non-metallic amorphous material which is thermodynamically an undercooled and frozen melt. Glass refers to a hard, brittle, transparent solid. Typical examples include soda-lime glass and borosilicate glass. A glass is an inorganic product of fusion which has been cooled to a rigid condition without crystallizing. Most glasses contain silica as their main component and a certain amount of glass former

"Glass-ceramic" means an inorganic non-metallic material where one or more crystalline phases are surrounded by a glassy phase so that the material comprises a glass material and a ceramic material in a combination or mixture. Thus, a glass ceramic is a material sharing many properties with both glass and more traditional crystalline ceramics. It is formed as a glass, and then made to crystallize partly by heat treatment.

The space between the crystallites is filled by the glassy matrix. Glass ceramics mainly refer to a mixture of lithium-, silicon-, and aluminium-oxides.

"Ceramic" means an inorganic non-metallic material that is produced by application of heat. Ceramics are usually hard and brittle and, in contrast to glasses or glass ceramics, display an essentially purely crystalline structure.

A zirconia containing dental ceramic article or framework is classified as "presintered" if the dental article of framework has been treated with heat (temperature range from about 900 to about 1100°C) for about 1 to about 3 hours to such an extend that the raw breaking resistance (Weibull strength Sigma 0) of the dental ceramic article or framework is within a range of about 15 to about 55 MPa or about 30 to about 50 MPa (measured according to the "punch on three ball test" (biaxial flexural strength) described in DIN EN ISO 6872, edition March 1999, with the following modifications: diameter of steel ball: 6 mm; diameter of support circle: 14 mm; diameter of flat punch: 3.6 mm; diameter of sample disc: 25 mm, thickness of sample disc: 2 mm; no grinding and polishing of samples.).

A presintered dental ceramic article or framework typically has a porous structure and its density (usually 3.0 g/cm³ for an Yttrium stabilized ZrO₂ ceramic) is less compared to a completely sintered dental ceramic framework (usually 6.1 g/cm³ for an Yttrium stabilized ZrO₂ ceramic). The diameter of the pores can be in a range of about 50 nm to about 150 nm (corresponding to about 500 to about 1500 Ǻ). A typical pore diameter is about 120 nm.

In contrast to this, presintering of a glass or glass ceramic material is typically effected in a temperature range of about 500 to about 750 °C.

The terms "sintering" or "firing" are used interchangeably.

A presintered ceramic framework shrinks during a sintering step, that is, if an adequate temperature is applied. The sintering temperature to be applied depends on the ceramic material chosen. For zirconia (i.e. ZrO₂) based ceramics a typical sintering temperature range is about 1200 °C to about 1500 °C. Al₂O₃ based ceramics are typically sintered in a temperature range of about 1300 °C to about 1700 °C. Glass ceramic materials are typically sintered in a range of about 700 to about 1100 °C for about 1 to about 3 hours.

A ceramic article is classified as "presintered" if the ceramic article has been treated with heat (temperature range from about 900 to about 1100°C) for about 1 to about 3 h to such an extend that the raw breaking resistance of the dental ceramic article is within a range of about 5 to about 55 MPa or about 5 to about 30 MPa (measured according to the "punch on three ball test" (biaxial flexural strength) described in DIN EN ISO 6872, edition March 1999, with the following modifications: diameter of steel ball: 6 mm; diameter of support circle: 14 mm; diameter of flat punch: 3.6 mm; diameter of sample disc: 20 mm, thickness of sample disc: 2 mm; no grinding and polishing of samples.).

A presintered ceramic article typically has a porous structure and its density (usually 3.0 g/cm³ for an Yttrium stabilized ZrO₂ ceramic) is less compared to a completely sintered dental ceramic article (usually 6.1 g/cm³ for an Yttrium stabilized ZrO₂ ceramic). The diameter of the pores can be in a range of about 50 nm to about 150 nm (corresponding to about 500 to about 1500 Ǻ). A typical pore diameter is about 120 nm.

Sintering typically includes the densification of a porous material to a less porous material (or a material having less cells) having a higher density, in some cases sintering may also include changes of the material phase composition (for example, a partial conversion of an amorphous phase toward a crystalline phase).

"Density" means the ratio of mass to volume of an object. The unit of density is typically g/cm³. The density of an object can be calculated e.g. by determining its volume (e.g. by calculation or applying the Archimedes principle or method) and measuring its mass.

A "porous material" sometimes refers to a material comprising a partial volume that is formed by voids, pores, or cells in the technical field of ceramics. Accordingly an "open-celled" structure of a material sometimes is referred to as "open-porous" structure, and a "closed-celled" material structure sometimes is referred to as a "closed-porous" structure. It may also be found that instead of the term "cell" sometimes "pore" is used in this technical field.

The structure of a material as referred to in this specification may be categorized as "open-celled", "closed-celled" and "generally free of cells".

The term "open-celled" relates to an "open porosity" according to the mercury porosimetry as defined in DIN 66133. Typical values are between about 6% and about 35%, of between about 15% and about 35%, or between about 30% and about 35%.

The term "closed-celled" relates to a "closed porosity". Closed cells are those cells which are not accessible from the outside and cannot be infiltrated by gases under ambient conditions.

The unit "cells per mm^{2"} is related to the number of cells present on a cross section of the sample to be analysed. A suitable test method is given in DIN 13925.

The volume of a sample can be determined based on the overall outer dimensions of the sample. The density of the sample can be calculated from the measured sample volume and the sample mass. The total volume of glass ceramic material can be calculated from the mass of the sample and the density of the used material.

The total volume of cells in the sample was assumed to be the remainder of the sample volume (100% minus the total volume of material).

A dental ceramic framework is classified as "absorbent" if the dental ceramic framework is able to absorb a certain amount of a liquid, comparable to a sponge. The amount of liquid which can be absorbed depends e.g. on the chemical nature of the dental ceramic framework, the viscosity of the solvent, the porosity and pore volume of the dental ceramic framework. E.g. a pre-sintered dental ceramic article, that is an article which has not been sintered to full density, is able to absorb a certain amount of liquid. Absorbing of liquids is typically only possible if the article has an open-porous structure.

By "dental mill blank" is meant a solid block (3-dim article) of material from which a dental article, dental workpiece, dental support structure or dental restoration can be machined. A dental mill blank may have a size of about 20 mm to about 30 mm in two dimensions, for example may have a diameter in that range, and may be of a certain length in a third dimension. A blank for making a single crown may have a length of about 15 mm to about 30 mm, and a blank for making bridges may have a length of about 40 mm to about 80 mm. A typical size of a blank as it is used for making a single crown has a diameter of about 24 mm and a length of about 19 mm. Further, a typical size of a blank as it is used for making bridges has a diameter of about 24 mm and a length of about 58 mm. Besides the above mentioned dimensions, a dental mill blank may also have the shape of a cube, a cylinder or a cuboid. Larger mill blanks may be advantageous if more than one crown or bridge should be manufactured out of one blank. For these cases, the diameter or length of a cylindric or cuboid shaped mill blank may be in a range of about 100 to about 200 mm, with a thickness being in the range of about 10 to about 30 mm.

By "machining" is meant milling, grinding, cutting, carving, or shaping a material having a 3-dim. structure or shape by a machine. Milling is usually faster and more cost effective than grinding.

A "green body" means an un-sintered ceramic item.

A "particle" means a substance being a solid having a shape which can be geometrically determined. The shape can be regular or irregular. Particles can typically be analysed with respect to e.g. grain size and grain size distribution.

A "powder" means a dry, bulk solid composed of a large number of very fine particles that may flow freely when shaken or tilted.

"Casting" means a manufacturing process by which a liquid or viscous material (e.g. solution or dispersion) is poured into a mould, which contains a hollow cavity of the desired shape, and then allowed to solidify.

The term "showing a light emission" within a certain range means that at least one emission band within the range of visible light can be found. The article emits more light than it is illuminated with. This does not necessarily exclude the presence of further emission bands outside this particular range.

The term "showing a light absorption" within a certain range means that at least one absorption band within the range of visible light can be found. The article emits less light than it is illuminated with. This does not necessarily exclude the presence of further absorption bands outside this particular range.

A "hardenable compound" is any compound which can be cured, polymerized or solidified e.g. by chemical crosslinking through radiation-induced polymerization, crosslinking by using an initiator or heating. Hardenable compounds often comprise one or more (meth)acrylate groups.

An "initiator" is a substance being able to start the hardening process of a hardenable compound.

"Ambient conditions" invention mean the conditions which the inventive solution is usually subjected to during storage and handling. Ambient conditions may, for example, be a pressure of about 900 to about 1100 mbar, a temperature of about -10 to about 60 °C and a relative humidity of about 10 to about 100 %. In the laboratory ambient conditions are adjusted to about 23 °C and about 1013 mbar.

A composition or solution is "essentially or substantially free of" a certain component within the meaning of the invention, if the composition or solution does not contain said component as an essential feature. Thus, said component is not wilfully added to the composition or solution either as such or in combination with other components or ingredient of other components. Ideally the composition or solution does not contain the said component at all. However, sometimes the presence of a small amount of the said component (e.g. up to about 0.1 or up to about 0.01 or up to about 0.001 wt% with respect to the weight of the dental article) is not avoidable e.g. due to impurities contained in the raw materials used.

As used herein, "a", "an", "the", "at least one" and "one or more" are used interchangeably. The terms "comprises" or "contains" and variations thereof do not have a limiting meaning where these terms appear in the description and claims.

Also herein, the recitations of numerical ranges by endpoints include all numbers subsumed within that range (e.g., 1 to 5 includes 1, 1.5, 2, 2.75, 3, 3.80, 4, 5, etc.).

If reference is made to a certain value of e.g. mol% or wt%, the calculation is based on the amount of oxide of the element referred to (unless specified otherwise).

### Brief Description of Figures

Figure 1 shows a flow chart of a possible production route by casting.
Figure 2 shows a flow chart of a possible production route by pressing and firing.
Figure 3 shows a flow chart of another possible production route using a colouring liquid.

### Detailed Description of the Invention

The natural fluorescence inherent to human dentin is a property that ceramic restorations based on zirconium oxide were not able to match until now. Instead, fluorescence, if at all, was provided by coating glassy veneer compositions containing fluorescent components on the surface of zirconium oxide based support structures. However, this approach did not produce restorations that "shine from within".

In order to adapt a restoration regarding its optical appearance to the surrounding teeth, the brightness of the restoration should be adjusted as exact as possible to that of the natural teeth. For this reason, some tooth color systems like the Vita™ 3D master system are based on brightness values. A very simple method to determine if a restoration fits from its brightness to the natural teeth is to take a black and white photograph (e.g. panchromatic film) of the dental restoration in the mouth of a patient. If the dental restoration cannot be distinguished from the teeth the brightness is typically correctly adapted.

The invention provides a dental article comprising a brighter material which can be used for producing more aesthetic dental support structures and final dental restorations.

Dental restorations can be provided having a higher irradiance. The property "irradiance" can be assessed e.g. by determining especially the L* value of the L*a*b* values and/or opacity of the respective material.

It was found that the inventive dental article emits more light than comparable articles without a fluorescing section (e.g. a fluorescent dental support structure). A dental restoration made according to the invention typically displays a superior irradiance.

As a result, the dental practitioner is now able to use a more translucent veneering material (and/or an increased layer thickness, if desired) without running the risk of reducing the overall brilliance of the final dental restoration, but with the benefit of better matching the translucency of natural teeth.

The dental practitioner can now also realize thin veneerings (e.g. in the form of a glaze, shade or painting), e.g. veneerings having a layer thickness in the range of about 1 to about 30 µm or of about 2 to about 20 µm or about 5 to about 15 µm without jeopardizing the brightness of the final dental restoration.

This enables the practitioner to produce dental restorations out of a single block without the need for additional layers of veneering material. This is also sometimes referred to as the manufacturing of "individualized monolithic zirconia restoration".

Fluorescent zirconium oxide containing material does not only match the behaviour of natural dentin in a dark environment with UV light present. It also facilitates the provision of dental restorations with a more natural, shining look in broad daylight. The desired natural look is produced inside the dental restoration through fluorescence of the zirconium oxide material used.

The inventive dental article has an upper (sometimes also referred to as "outer surface") and a lower surface (sometimes also referred to as "inner surface"). The dental article comprises two sections, section A and section B.

Ideally, the shape provided by the lower surface of the dental article corresponds essentially to or is proportionally enlarged to the shape of the (prepared) tooth or teeth or abutment of an implant or situation in the mouth of the patient. Some portions of the inner surface are typically concave.

The shape of the upper surface of the dental support structure is typically convex.

Usually, the lower surface of the dental article essentially corresponds to the lower surface of section A. The upper surface of section A typically corresponds to the lower surface of section B, if section B has a (e.g. geometrically defined) 3-dim shape.

Section A can also be referred to as support or substructure of the dental article. The support structure typically provides the strength and toughness necessary for dental restorations.

If the material of section A has been sintered to its final stage, the material of section A typically fulfils at least one of the following physical parameters:
- breaking resistance: at least about 400 MPa, or at least about 700 MPa or at least about 1000 MPa,
- density: from about 5.9 to about 6.1 g/cm³ or from about 6.0 to about 6.1 g/cm³, and/or
- light emission, in particular, fluorescence emission with bands in the region of the visible light.(Dy: bands between 470 and 510 nm and between 565 and 615 nm; Sm: multiple bands between 550 and 725 nm; Eu: multiple bands between 580 and 725 nm).

If desired, these parameters can be measured as follows:
The breaking resistance of the sintered dental ceramic article can be determined according to the "punch on three ball test" (biaxial flexural strength) described in DIN EN ISO 6872, edition March 1999, with the following modifications: diameter of steel ball: 3 mm; diameter of support circle: 12 mm; diameter of flat punch: 3.6 mm; diameter of sample disc: 16 mm, thickness of sample disc: 1.6 mm (+/- 0.05 mm); grinding of samples with 10 µm disc to be +/- 0.05 mm plan parallel and polishing of samples consecutively with 9 and 3 µm.

The density can be obtained from determining the mass (by weighing) and the volume (e.g. by calculation or using the "Archimedes Method").

The fluorescence properties can be determined using an optical setup comprising the following parts: GC America G-Light as light source, irradiating light of around 409 nm wavelength, an Ulbricht sphere, fiber optics from Topsensor Systems as light conductor and an A/D converter. A sample having the shape of a disc (diameter of 16 mm, thickness of 1.6 mm) can be used to cover the opening of the Ulbricht sphere. The light emission spectrum of the sample can be measured while transilluminating it with exitation radiation (violet light). Excitation radiation of shorter wavelengths is also suited for fluorescence measurements.

Section A comprises zirconia (zirconium oxide) in an amount of at least about 80mol% or at least 85 mol% or at least 90 mol% or at least 95 mol%, with respect to the total amount of section A.

Section A can contain zirconia in an amount up to about 98 mol% or up to about 97 mol% or up to about 95 mol% or up to about 92 mol% with respect to the total amount of section A.

Useful ranges for zirconium oxide contained in section A of the dental article include from about 80 to about 98 mol% or from about 85 to about 97 mol% or from about 90 to about 95 mol% with respect to the total amount of section A.

That is, section A comprises or essentially consists of zirconia, stabilizer(s) and further additives.

Components containing zirconium oxide are commercially available from e.g. Tosoh Corp., Japan, Toray, Japan or Inframat Corp., USA.

Zirconium oxide containing materials which can be used are described also in US 6,713,421 and US 2004/0119180, the contents of which with respect to the description of zirconium oxide containing materials are herewith incorporated by reference.

Section A also comprises a stabilizer.

Components which can be used as stabilizers include Y, Mg, Ca, Ce, La combinations and mixtures thereof.

Using e.g. yttrium stabilized zirconia can be advantageous due to its ability to stabilize certain crystal structures of the zirconia material and thus enabling the practitioner to provide .materials with high strengths.

The stabilizer can be present in section A of the dental article in an amount of about 1 to about 8 mol% or of about 1.5 to about 6 mol% or of about 2 to about 5 mol%.

In the sintered stage, the stabilizer(s) are typically present as oxides of the respective elements.

Section A also comprises a component X comprising Dy or Sm or Eu or mixtures or combinations thereof. Thus, section A might comprise as component X either Dy or Sm or Eu, or the combinations a) Dy and Sm, b) Dy and Eu, c) Sm and Eu or d) Dy, Sm and Eu.

Adding component X to zirconia typically results in an increased light emission (especially fluorescence emission) in the range of the visible spectrum of light.(e.g. about 400 to about 800 nm).

Section A can contain component X in an amount of at least about 0.1 mol% or at least about 0.2 mol% or at least about 0.5 mol% (calculation based on the amount of oxide of the element referred to).

Section A can contain component X in an amount up to about 5 mol% or up to about 3 mol% or up to about 2 mol% (calculation based on the amount of oxide of the element referred to). Useful ranges for component X include from about 0.1 to about 5 mol% or from about 0.2 to about 3 mol% or from about 0.5 to about 2 mol% (calculation based on the amount of oxide of the element referred to).

Alternatively or in addition the amount of component X can also be given in wt% based on the oxide (in this case, substance X has an oxidation number of III).

Thus, section A can contain component X in an amount of at least about 0.3 wt% or at least about 0.6 wt% or at least about 1.5 wt%.

Section A can contain component X in an amount up to about 15 wt% or up to about 9 wt% or up to about 6 wt%.

Useful ranges for component X contained in section A of the dental article include from about 0.3 wt% to about 15 wt% or from about 0.6 wt% to about 9 wt% or from about 1.5 wt% to about 6 wt%.

If the concentration is outside the above mentioned ranges, the desired fluorescence or light emission intensity sometimes cannot be achieved or the mechanical properties of the material may change disadvantageously. In particular, if the concentration is above a value of about 5 mol%, then quenching, i.e. a decrease in fluorescence may occur. On the other hand, if the concentration is below a value of about 0.1 mol%, then fluorescence might be too weak to produce a visible or sufficient effect.

Components containing dysprosium, samarium or europium are commercially available from e.g. Aldrich, USA or Fluka, Germany.

Dysprosium, samarium or europium containing components can be provided as oxides or water soluble compositions including acetate, citrate, carbonate, chloride and nitrate.

The use of salts or oxides which do not produce corrosive side products upon heating are preferred. Preferred salts include dysprosium and samarium acetate and carbonate.

The amount of stabilizer and component X being present in section A of the dental article is typically in a range of about 2 to about 10 mol% or in a range of about 3 to about 8 mol%, the amount being calculated with respect to the whole composition of section A.

If the amount is outside this range, the desired physical features like breaking strength and desired intensity of fluorescence may not be obtained.

Besides zirconium oxide, the dental article can comprise further ceramic oxides including hafnium oxide or alumina, if desired.

If present, those oxides are typically present in an amount of about 1 to about 5 mol% or of 2 to about 3 mol% with respect to section A.

Besides component X, section A can contain further additives, including further colouring additives.

Further additives which can be present include erbium (Er), iron (Fe), praseodymium (Pr), manganese (Mn) and combinations thereof.

There is no need for additives to be present, however, if present they are typically present in an amount of up to about 1 mol% or up to about 0.1 mol% or up to about 0.01 mol%.

Typical ranges include from about 0.0001 mol% to about 1 mol% or from about 0.001 mol% to about 0.8 mol%.

The additives can be added to the zirconium oxide powder before conducting a pressing and/or pre-sintering step or can be added afterwards, e.g. after a presintering step but before a final sintering step. The additives can be added either as a solid substance or applied to a pre-sintered article using a colouring solution or colouring composition, e.g. by dipping the article into a composition comprising the desired additive or by applying the composition comprising the desired additive on the surface of the article (e.g. by brushing or spraying). A suitable processes is also described e.g. in US 6,709,694 or EP 1 486 476 A1. The disclosure of these documents is herewith incorporated by reference.

According to a particular embodiment section A of the dental article comprises (in the sintered ceramic, all components are present as oxides):

| | |
|---|---|
| ZrO₂: | from about 80 mol% to about 98 mol% or from about 85 mol% to about 97 mol%, |
| Stabilizer: | from about 1 mol% to about 8 mol% or from about 1.5 mol% to about 6 mol% (especially if the stabilizer comprises Y), |
| Component X: | from about 0.1 mol% to about 5 mol% or from about 0.5 mol% to about 2 mol% (especially if the stabilizer comprises Dy), |
| Additives: | (e.g. colouring substances, temporary binders, buffers and/or thixotropic substances) from about 0.0001 mol% to about 1 mol% or from about 0.001 mol% to about 0.8 mol%, wherein the term additive includes components other than ZrO₂ Y₂O₃, Dy₂O₃, Sm₂O₃ or Eu₂O₃. |

If section A of the dental article has the above composition, it can be especially suitable for producing a high aesthetic dental restoration or part thereof and is especially suitable to be used as a dental support structure. The final article then has a typically tooth colour and/or can take shades of yellow and brown if further colouring additives are present and it mimicries the appearance of natural human or animal dentin.

According to a further embodiment section A comprises a crystalline phase. The crystalline phase typically comprises at least two different crystal structures.

According to certain embodiments, section A of the dental article does typically not comprise a glass, glass ceramic material or glass ionomer cement in an amount above about 5 wt% or above about 3 wt % or above about 1 wt% or above about 0.5 wt% with respect to the total amount of section A.

According to certain embodiments, section A of the dental article does also typically not comprise components selected from Nd, Cu, Cr, Ti, or mixtures thereof in an amount above about 0.012 wt% or above about 0.010 wt% or above about 0.008 wt% or above about 0.005 wt% with respect to the total amount of section A.

That is, certain embodiments of the section A of the dental article are essentially free of these materials or components, especially as regards Nd and/or Ti.

The presence of a glass, glass ceramic material in section A in combination with zirconia may lead to a reduced strength of the final product. This is often not desirable.

Moreover, without wishing to be bound to a certain theory, the presence of components like Nd, Cu and/or Cr in section A in combination with zirconia and component X as described in the present text, might influence the overall emission properties of section A. That is, the intensity of the fluorescence of section A as desired might not be obtained to the full extent.

The material of section B of the dental article is located at least partially above or on top of the outer or upper surface of section A.

The material of section B may be present in one or more (e.g. 2 to about 5) comparably thin layer(s).

A typical layer thickness is from about 0.01 mm to about 3 mm or from about 0.1 mm to about 2.5 mm. The thickness of the layer may vary. Thus, there may be parts of the surface where the thickness of the layer is very thin (e.g. in a range from about 0.01 to about 0.5 mm) and there may be parts of the surface where the thickness of the layer is thicker (e.g. in a range from about 1 to about 3 mm).

The thickness of the layer of the material applied on at least a portion of the surface of section A should, however, not be too high. If the layer thickness is greater than the above mentioned values, the overall aesthetic appearance of the final dental restoration might be negatively influenced. The desired "shine from within" effect of section A might not be visible anymore. Additionally, a very high thickness of section B might have a negative effect on the mechanical stability of the dental article.

The Material being contained in section B is not only different from the material contained in section A with respect to its chemical nature or composition. It typically also differs with respect to its physical parameters.

The material used in section B may comprise a glass or glass ceramic. The material can typically be characterized by at least one of the following parameters:
- bending strength (determined according to ISO 6872:2008): from about 50 to about 400 MPa or from about 70 to about 300 MPa;
- opacity: from about 20 to about 90% or from about 30 to about 70%.

If desired, the opacity can be determined with a Hunterlab color measuring device Labscan 5100.

Typically the translucency is specified by the opacity of a material relative to daylight.

Typical ranges of the opacity of section B also include ranges from about 40% to about 90% or from about 45% to about 85% or from about 50 to about 80%. For comparison, natural dental enamel may have an opacity of about 50% to about 60%, natural dentine may have an opacity of about 60% to about 80%, natural opaque dentin may have an opacity of about 80% to about 90%.

The value of the opacity is typically influenced by the thickness of the sample analysed. The above values refer to samples having a thickness of about 1.5 mm.

Glass or glass ceramics materials which can be used can be characterized by at least one of the following features:
- comprising: 55 wt% to 75 wt% of silicon oxide, 8 wt% to 22 wt% of aluminum oxide, 0 wt% to 8 wt% of boron oxide, 3 wt% to 12 wt% of potassium oxide, 4 wt% to 12 wt% of sodium oxide, 0 wt% to 2 wt% of cerium oxide, 0 wt% to 2 wt% of tin oxide, 0 wt% to 3 wt% of zinc oxide, 0 wt% to 4 wt% of phosphor oxide, 0 wt% to 3 wt% of calcium oxide, 0 wt% to 3 wt% of lithium oxide, and 0 wt% to 1 wt% of fluoride, 0 wt% to 3 wt.% of lanthanum oxide or lanthanide oxide.
- Coefficient of thermal expansion: about 8 * 10⁻⁶K⁻¹ to about 15.8 * 10⁻⁶K⁻¹ or 8 * 10⁻⁶K⁻¹ to about 9 * 10⁻⁶K⁻¹ or about 12 * 10⁻⁶K⁻¹ to about 13.6 * 10⁻⁶K⁻¹ or from about 15 * 10⁻⁶K⁻¹ to 15.8 * 10⁻⁶K⁻¹.
- mean particle size: range from about 1 µm to about 60 µm, or from about 5 to about 40 µm (measured with laser diffraction);
- melting temperature (range): around or less than about 1000 °C
- density: about 2.0 to about 2.8 or about 2.2 to about 2.6 g/cm³ (according to the technical data sheet provided by the manufacturer) and/or
- glass transition temperature: about 500 to about 600 °C or about 520 to about 580 °C, preferably about 550 °C.

If desired, the mean particle size can be determined using a commercially available granulometer (Laser Diffraction Particle Size Analysis Instrument, CILAS 1064; CILAS Comp.) according to the instruction of use provided by the manufacturer.

The glass or glass ceramic material is preferably selected to be compatible for use in human bodies. Furthermore, the glass ceramic material is preferably selected to provide good aesthetic appearance for the dental restoration.

Glass or glass ceramic materials which may, for example, be used are available under the designations:
- "VMK 95" and "Omega 900" from Vita Zahnfabrik, Bad Säckingen, Germany;
- "IPS Classic" and "IPS d.Sign" from Ivoclar Vivadent, Liechtenstein;
- "Vintage" from Shofu, Japan; and
- "REFLEX" from Wieland GmbH &Co.KG, Pforzheim, Germany.
- "VM9" from Vita Zahnfabrik, Bad Säckingen, Germany
- "LAVA Ceram" from 3M ESPE, Seefeld, Germany
- "Cerabien Zr" from Noritake, Japan
- "LM ZrO2" from Chemichl AG, Liechtenstein.

The crystalline phase of the glass ceramic material may also comprise leucite, lithium disilicate, zirconia and/or mica crystallites. Suitable lithium silicate glass ceramic materials are described e.g. in US 7,452,836 B2, US 6,306,784 B1, US 6,121,175. These references are herewith incorporated by reference. A typical composition comprises: SiO₂ in an amount of about 55 to about 71 wt%, Al₂O₃ in an amount of about 5 to about 16 wt%, B₂O₃, in an amount of about 0.2 to about 10 wt%, K₂O in an amount of about 4 to about 10 wt%, Na₂O in an amount of about 3 to about 14 wt% and optionally further components selected from CaO, F, P₂O₃, Li₂O, BaO and ZnO.

The glass and/or glass ceramic material does typically not comprise so-called reactive glass(es), that is, glass(es) which can undergo a neutralization or ion-exchange reaction with acidic substances. Reactive glasses are typically used in glass ionomer cement (GIZ) compositions.

A cement reaction is defined as the reaction of a reactive glass with a polyacid, which results in a hardened product typically within utmost 20 min upon mixing.

Preferred glasses and/or glass ceramic materials to be used according to the present invention comply with the requirements according to EN ISO 6872:2008. In contrast to reactive glasses used in glass ionomer cements, the glasses and/or glass ceramic materials to be used according to the present invention have a reduced solubility in diluted acetic acid, e.g. less than about 100 µg/cm² (measured according to EN ISO 6872:2008, Table 1, Chapter 7.6).

The material of section B of the dental article can also be provided as an article having a geometrically defined 3-dim. structure or shape (e.g. as a machined dental veneering or facing).

The term "dental facing" includes sintered dental facings and dental facing precursors (not sintered to final density yet). A dental facing typically has an upper and a lower surface, wherein the lower surface typically has a concave shape.

A dental facing precursor for example can be characterized by one or more of the following features:
a. comprising an open-celled material,
b. comprising a glass and/or glass ceramic material,
c. being proportionally enlarged relative to a sintered facing by a magnification factor of between 1.12 to 1.9,
d. coefficient of thermal expansion: from about 8 * 10⁻⁶K⁻¹ to about 15.8 * 10⁻⁶K⁻¹,
e. the material density being in a range of from about 0.6 g/cm³ to about 2.5 g/cm³.

The facing precursor typically has a shape that corresponds to a proportional enlargement of the final facing with a magnification factor of preferably between about 1.05 and about 2.2, in more particular between about 1.05 and about 1.8, more preferably between about 1.12 and about 1.5.

The facing precursor typically comprises an inner surface corresponding to a proportionally dimensioned counter-surface of an outer surface of a frame. The inner surface of the facing precursor may be scaled so that it matches the outer surface of the frame when the facing precursor has been sintered.

During sintering, the material of the facing precursor typically shrinks toward the inner surface of the facing precursor with the inner surface generally maintaining its shape.

The facing precursor is typically produced by machining a blank comprising a glass or glass ceramic containing material.

Machining the facing precursor for making a facing, typically comprises the step of controlling a CNC machine based on data related to the shape of the facing precursor which are obtained from a CAD system, wherein the facing precursor is proportionally enlarged with respect to the facing.

The material the facing is made of is typically selected such that a certain translucency as described above can be provided.

Composite materials which can be used are typically polymerizable or hardenable compositions. Compositions comprising components bearing (meth)acrylate groups were found to be useful. These compositions typically also comprise fillers (including silica materials). Some of these compositions are also commercially available (e.g. Sinfony^{™} Light Curing Composite, 3M ESPE).

Like the material of section A, the material of section B does typically not comprise certain components like glass ionomer cement, Nd, Cu or combinations and mixtures thereof. Thus, with respect to certain embodiments, the respective description for the material of section A above may also apply to the material for section B.

Thus, according to a specific embodiment, section B does not comprise e.g. Nd.

Section A of the dental article can be produced using different methods.

One embodiment (Option A) is directed to a process comprising the steps of
a) providing a composition comprising zirconium oxide powder, a liquid, a stabilizer and component X,
b) casting the composition in a mould,
c) drying the composition (e.g. to obtain a green body),
d) optionally presintering the composition of step b) or c),
e) optionally machining the green body obtained in step b), c) or the pre-sintered article obtained in step d), and
f) optionally firing the article from step b), step c) step d) or step e).

Liquids which can be used include water and low boiling alcohols (e.g. methanol, ethanol, and propanol) and ketons (e.g. acetone).

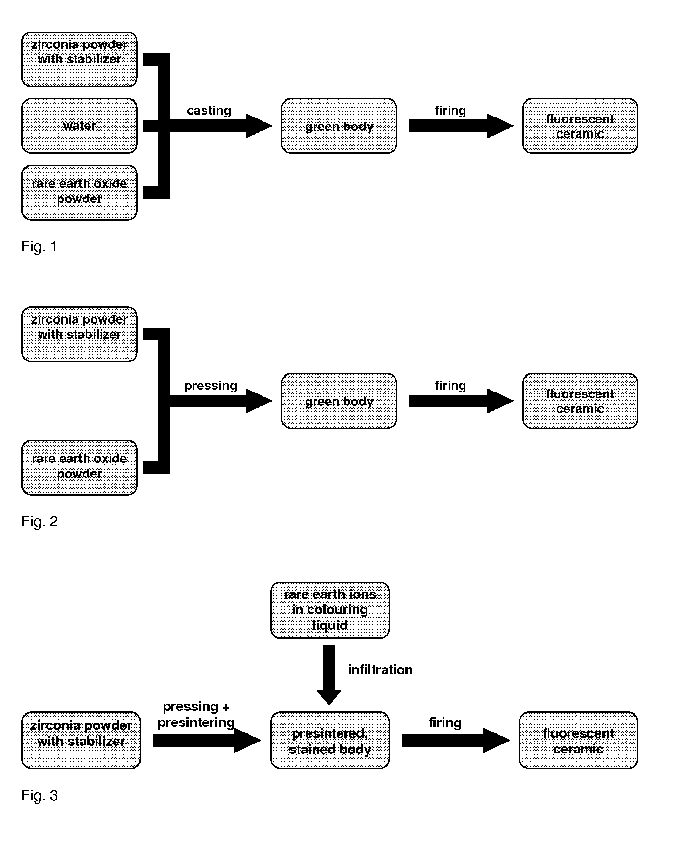

This process is exemplified in Fig. 1. Fig. 1 is a flow chart showing a production route using a casting step.

Drying, if desired, is typically carried out within a range from about 30 to about 120°C.

Presintering, if desired, is typically carried out within a range from about 700 to about 1100 °C.

Another embodiment (Option B) is directed to a process comprising the steps of
a) providing a composition comprising zirconium oxide, a stabilizer and component X, e.g. by mixing the zirconium oxide already containing a stabilizer with component X being provided as an oxide of the respective element,
b) applying pressure and/or temperature to the composition to obtain a 3-dim article (e.g. green body),
c) optionally machining the 3-dim article, and
d) optionally firing the 3-dim article.

This process is exemplified in Fig. 2. Fig. 2 shows a flow chart of possible production route comprising a pressing step.

The individual components used in step a) are typically provided in a solid form, e.g. as a powder.

Typical granule sizes for the powder include from about 5 to about 500 µm or from about 20 to about 200 µm. If desired, the grain size can be determined as described above.

The pressure applied is typically within a range from about 10 to about 1000 MPa or from about 20 to about 500 MPa.

Another embodiment (Option C) is directed to a process comprising the steps of
a) providing a composition comprising zirconium oxide and a stabilizer,
b) applying pressure and/or temperature to the composition to obtain a 3-dim article (e.g. green body),
c) optionally machining the 3-dim article,
d) treating the the 3-dim article with a composition comprising component X being selected from Dy or Sm or Eu or mixtures thereof,
e) optionally firing the 3-dim article,

This process is exemplified in Fig. 3. Fig. 3 shows a flow chart of possible production route comprising the use of a colouring liquid.

A further embodiment is directed to a process for producing the dental article as described in the text.

The process typically comprises the following process steps:
a) providing a ceramic article comprising
   zirconia in an amount of at least about 85 mol%,
   a stabilizer and
   a component X comprising Dy or Sm or Eu or mixtures thereof,
a') optionally machining the ceramic article (e.g. in order to obtain a dental ceramic article or a dental ceramic support structure with an upper and a lower surface),
a") optionally treating the machined article or at least a portion of the machined article with a colouring solution,
a''') optionally sintering the machined article,
b) covering at least a part of the surface of the article with a glass, glass ceramic or composite material or applying a glass, glass ceramic or composite material to at least a part of the surface of the article,
c) optionally firing the article the surface of which has at least been partially covered with a glass or glass ceramic material.

The article of step a) can be fully sintered or can be in a presintered stage.

If the ceramic article is in a presintered stage, the article has typically a density being below the density of the article in the final sintered stage. A ceramic article in the presintered stage typically also has a porous structure, which allows the penetration of further substances into the pores of the ceramic material, if desired. Thus, the presintered material may have an open-celled structure. The presintered material may also be characterized having absorbent properties.

In order to avoid a negative influence on the crystal structure of the ceramic article, it can however be beneficial, if the ceramic article is already present in the fully sintered stage before the glass, glass ceramic or composite material of step b) is applied.

Sintering conditions are dependant on the material used. An oven which can be used is the commercially available LAVA™ Therm (3M ESPE; Germany). During the sintering process the dental ceramic article is sintered to its final shape, thereby undergoing changes with regard to dimension, density, hardness, raw breaking resistance and/or grain size.

The sintering usually takes place for a ZrO₂ based ceramic at a temperature above about 1300°C, preferably above about 1400°C, more preferably above about 1450°C and lasts for at least about 0.5 h, preferably for at least about 1 h, more preferably for at least about 2 h.

Generally, the sintering conditions are adjusted such that the sintered dental article has a density of equal or greater than about 98 % compared with the theoretically achievable density. In one embodiment this can be accomplished using a temperature above about 1300 °C.

It is, however, also possible, that before sintering the ceramic article to final density and before covering the surface of the article as described in step b), the ceramic article is treated with a further composition. Such a composition can be a colouring liquid comprising colouring ions which are typically different from the ions which may already be present in the ceramic article causing the fluorescence.

Preferably, the composition should have an adequate viscosity so that sufficient wetting and colouring of and penetration into the pores of the zirconium oxide containing article can be achieved. Good results can be obtained with a solution having a dynamic viscosity of about 1 mPa*s up to about 100 mPa*s or up to about 80 mPa*s or up to about 60 mPa*s.

If desired, the dynamic viscosity can be determined with a Physica MCR301 instrument using a cone plate geometry, diameter 50 mm, angle (cone) 1°, at 23 °C.

A typical shear rate is 200 rounds/s, however, generally the viscosity of liquids is independent from the shear rate in a wide range.

If the viscosity of the composition to be applied in this process is too low, the colour of the coloured dental article might not be homogenous.

Colouring liquids which can be used include those described in US 6,709,694. This document is herewith incorporated by reference.

According to a further embodiment, the above described process comprises a step wherein at least part of the surface of the ceramic article is treated with a composition comprising Eu.

This process step is typically carried out before step b) is applied. The composition comprising Eu is typically applied to at least part of the surface of the ceramic article in the presintered stage.

Especially the use of a composition comprising Eu as a so-called "liner" has been proven to be advantageous for the production of highly aesthetic dental restorations. In this respect the composition is typically applied only on certain portions of the surface of the ceramic article. These portions include in particular the edges of the article, close to the preparation line.

The application of the colouring liquid or of the composition comprising Eu to at least a portion of the surface of the ceramic article can be done by dipping, spraying, brushing or painting. If a precise application is desired, using a brush can be preferred.

If applied, the article is usually treated with the colouring composition for about 0.1 to about 5 min, preferably from about 1 to about 3 min at room temperature (about 23 °C). Preferably no pressure is used.

Drying the coloured article is not necessary, but can be preferred to reduce the time needed for firing and to avoid undesired inhomogenous colour effects. Drying can be effected by simply storing the dental ceramic article on a surface at ambient conditions for a couple of hours (about 1 to about 3 h).

The glass, glass ceramic or composite material is typically applied to only a part of the surface of the ceramic article. If the ceramic article has the shape of support structure of a dental crown or has already the shape of a dental crown, the material is typically only applied on the outer surface of the article.

The material can have the consistency of a slurry (e.g. powder/liquid mixture) or may already have a 3-dim. shape (e.g. the shape of a dental veneer or facing) or might be applied by over-pressing with a viscous glass melt.

The application can be done by brushing the material on the surface (e.g. with the aid of a brush, spatula or all means which allow for an application of the composition to small objects especially those having concave and/or convex surfaces), dipping the article into a slurry of the material or by placing the material having a 3-dim. shape on the surface of the ceramic article.

A typical layer thickness is within a range from about 0.01 to about 3 mm or from about 0.1 to about 2.5 mm.

The above described method of producing a dental article may further comprise the following step (optional): aplying a stain or glacing composition on the outer surface of the sintered facing.

Glazing the facing may be advantageous to achieve a good optical appearance of the dental restoration and/or to render the dental restoration more durable.

The invention is also directed to a kit of parts comprising part 1 and part 2.

Part 1 typically comprises a blank (or more specifically, a dental mill blank). The blank typically comprises zirconia in an amount of at least about 85 mol%, a stabilizer and a component X being selected from Dy or Sm or Eu or mixtures thereof as described in the present text.

Part 2 typically comprises a glass, glass ceramic or composite material as described in the present text.

The blank is used for the production of a dental article as described in the present text.

The material of the blank is typically in a presintered stage or in a stage which allows the blank to be machined. Thus, the blank should have a sufficient raw breaking resistance. This stage is sometimes also called "green body". That is, the material may have already been slightly fired to a certain temperature to increase the raw breaking resistance of the material.

Presintering, if desired can be carried out in a temperature range from about 700 °C to about 1100 °C or from about 800 °C to about 1000 °C.

Thus, in certain embodiments the blank (before conducting a final sintering or firing step) can typically be characterized by at least one or more of the following features:
- raw breaking resistance: from about 5 to about 55 MPa, or from about 5 to about 30 MPa,
- density: from about 2.8 to about 3.5 g/cm³, or from about 2.9 to about 3.1 g/cm³.

The raw breaking resistance can be determined according to DIN EN ISO 6872 (with the following modifications: diameter of steel ball: 6 mm; diameter of support circle: 14 mm; diameter of flat punch: 3.6 mm; diameter of sample disc: 20 mm, thickness of sample disc: 2 mm; no grinding and polishing of samples.).

A zirconium oxide containing blank can be produced by any standard procedure known to the person skilled in the art, including uniaxial pressing, cold isostatic pressing (CIP), and slip casting.

The blank is typically contained in a holding device like a frame or fixed on a stub. Holding devices including frames have been proven to be useful. Sometimes it can be desirable, if the blank is put in a magazine, either for storing or for machining. The holding device may facilitates the machining of the dental article, e.g. by a machining device such as a milling device. Examples of holding devices are shown in US 2003/0132539, US 6,769,912 and EP 0 455 854 B1. The content of these documents with regard to holding devices (e.g. frames and stubs or supporting body) is herewith incorporated by reference and regarded part of the text of the present invention.

Fixing of the dental article on a stub can be achieved e.g. by gluing. The fixing should be such that the dental article can be processed in a milling machine e.g. on a Cerec™ InLab machine available from Sirona AG, Bensheim, Germany.

The glass, glass ceramic or composite material is basically the same as described above, when describing the material being present in section B of the dental article. These materials are typically contained in containers. The material may be present as powder (especially for glass or glass ceramics) or as a paste (especially if a composite material is used). It is, however, also possible that the glass or glass ceramic is provided as a paste or slurry.

The container may have the shape of a bottle, (screw) tube, flasks, syringe, capsule, cartridge and the like. It can also be preferred, if the composition is stored in a receptacle (e.g. a blister package) which also allows for using the composition as it is, that is, without the need for an additional mixing step.

The container can be a disposable container ("single use container").

The container is preferably sealed during storage and before use and re-sealable after use.

Sealing of the container can be accomplished e.g. by using removable caps, stoppers or foils, including self-adhesive foils.

The volume of the container is not particularly limited. Typically, the container has a volume in the range of about 0.1 to about 1 I or from about 0.5 ml to about 100 ml or from about 1 ml to about 10 ml.

The container typically contains a delivery system like a nozzle.

The kit may comprise a variety of different compositions e.g. in different volumes and/or shades. For example, the kit may comprise 1 container comprising an uncoloured or unpigmented composition and from about 1 to about 20 containers comprising coloured or pigmented compositions (e.g. compositions adapted to different tooth colours). The kit may also comprise at least 2 containers comprising different coloured or pigmented compositions.

A further embodiment is directed to the use of a blank (or dental mill blank) for producing a dental article as described in the present text.

The production step typically includes the step of machining the dental article and/or the blank.

A typical process of using the blank includes the steps of
- providing the blank (especially in a presintered stage),
- machining the blank (especially to a shape of a dental support structure),
- optionally sintering the machined blank,
- applying a glass, glass ceramic or composite material on at least a part of the surface of the machined blank,
- firing the blank containing a glass, glass ceramic or composite material.

After machining the blank to a desired structure, e.g. a dental support structure, the material is fired or sintered. Typical firing or sintering temperatures include a range from about 1200 °C to about 1500 °C.

The blank can be produced as described above with respect to the production of section A of the dental article.

According to one embodiment, component X can be added before production of a green body by mixing a zirconium oxide containing material (which may already contain the stabilizer) with component X or a composition or oxide containing component X.

Adding the component X from the very beginning, i.e. before the zirconium oxide containing material is pressed into a certain shape, often results in a very homogeneous distribution of the substances throughout the powder mixture.

According to another embodiment, component X can be added after a presintering step of a zirconium oxide containing material (which may already contain the stabilizer) e.g. by infiltrating the zirconium oxide containing material with a composition containing the desired ions.

Using the infiltration route can facilitate the whole production process e.g. by reducing the number of process steps needed for providing a coloured and fluorescent material.

The dental article of the present invention does typically not contain components or additives which jeopardize the intended purpose to be achieved with the present invention, i.e. providing an aesthetic dental restoration. Thus, components or additives added in an amount which finally results in a non-tooth-coloured article are usually not contained in the dental article. Typically, an article is characterized as not being tooth coloured if it cannot be allocated a colour from the Vita™ colour code system, known to the person skilled in the art. Additionally, components which reduce the mechanical strength of the dental restoration to a degree, where mechanical failure may occur, are also not included in the dental article.

Thus, for example certain embodiments of the dental article do typically not contain or are essentially free of either or more of the elements or ions of neodymium (Nd) and/or copper (Cu).

Moreover, the dental article does typically not comprise one or more of the following components selected from cross-linkable polyacid(s), fluoroalumina silicate glass(es) and mixtures thereof.

Cross-linkable polyacids are e.g. polyacrylic acid, polygalaturonic acid, polyethylene-co-maleic acid, that is, acids which can be obtained by polymerizing polymerizable acidic monomers. Usually, polyacids bear a huge number of acidic groups, typically at least one acidic group per polymerized acidic monomer.

The complete disclosures of the patents, patent documents, and publications cited herein are incorporated by reference in their entirety as if each were individually incorporated. The above specification, examples and data provide a description of the manufacture and use of the compositions and methods of the invention. The invention is not limited to the embodiments disclosed herein. One skilled in the art will appreciate that many alternative embodiments of the invention can be made without departing from the spirit and scope of thereof.

The following examples are given to illustrate, but not limit, the scope of this invention.

### Examples

Unless otherwise indicated, all parts and percentages are on a weight basis, all water is deionized water, and all molecular weights are weight average molecular weight. Moreover, unless otherwise indicated all experiments were conducted at ambient conditions (23°C; 1013 mbar).

The rare earth element containing compounds can be obtained e.g. from Aldrich, Merck or Fluka, Germany. According to the information provided by the manufacturer, TZ-3Y-SBC and TZ-3Y-SBE powders contain binder material in two different amounts, whereas TZ-3Y-E powder is essentially free of a binder material.

In Examples 1 to 3, 1.5 mol% of rare earth oxides (R₂O₃) were added to a zirconia material which already contained about 3.0 mol% of yttria (Y₂O₃).

### Example 1:

50 g of zirconia powder (TZ-3YSB-C from Tosoh) and 2.27 g dysprosia were mixed. The powder was pressed uniaxially into cylindric blocks of 20 mm diameter and 20 mm height at a pressure of about 200 MPa (manual lab press). The blocks were presintered at about 900 °C and cut into discs of 2 mm thickness and sintered in a furnace (Lava™ Therm, 3M ESPE) at about 1500 °C for 2 hours (heating 10°C/min).

Veneering glass powder (Correction 1, Lava DVS Kit, 3M ESPE) was pressed uniaxially into cylindric blocks of 20 mm diameter and 20 mm height at a pressure of about 5 bar (manual lab press). The blocks were presintered at about 620°C in a dental furnace under ambient atmosphere to a density of about 1.6 g/cm³ and cut into discs of 1 mm thickness with a diamond saw under water cooling.

A fully sintered zirconia disc was fused to a presintered veneering glass disc using an aqueous slurry of the Correction 1 powder and the modelling liquid (Lava™ DVS Kit, 3M ESPE) and fired at about 790 °C. The merged disc of 2.1 mm (1.6 mm zirconia support, 0.5mm veneering glass) was polished with a 3 µm diamond disc on both sides.

### Example 2:

The procedure of Example 1 was repeated. However, instead of 2.27 g of dysprosia, 2.12 g of samaria were added to the zirconia powder.

### Example 3:

4 g of zirconia powder (TZ-3YSB-C from Tosoh) and 0.17 g europia were mixed. The powder was pressed uniaxially into discs of 20 mm diameter and 2 mm height at a pressure of about 200 MPa (manual lab press). The discs were sintered in a furnace (Lava™ Therm, 3M ESPE) at about 1500 °C for 2 hours (heating 10°C/min).

Veneering glass powder (Correction 1, Lava™ DVS Kit, 3M ESPE) was pressed uniaxially into cylindric blocks of 20 mm diameter and 20 mm height at a pressure of about 5 bar (manual lab press).

The blocks were presintered at about 620°C in a dental furnace under ambient atmosphere to a density of about 1.6 g/cm³ and cut into discs of 1 mm thickness with a diamond saw under water cooling.

A fully sintered zirconia disc was fused to a presintered veneering glass disc using an aqueous slurry of the Correction 1 powder and the modelling liquid (Lava™ DVS Kit, 3M ESPE) and fired at about 790 °C. The fused disc was polished with a 3 µm diamond disc on both sides, resulting in an overall thickness of 2.1 mm (1.6 mm zirconia support, 0.5 mm veneering glass).

### Comparative Example:

The procedure of Example 1 was repeated. However, instead of 2.27 g of dysprosia, no additive was added to the zirconia powder.

The brightness of the veneered discs was determined by taking photographs of the discs against a black background under different illumination conditions (daylight, daylight + UV light, neon light + UV light). The camera used was a Canon PowerShot G10. The photographs were analysed regarding the brightness of the samples with Paint Shop Pro software (version 7.00) from Jasc Software. A representative portion of the disc (more than 50%) was marked and the average brightness of that section determined as arbitrary number.

**Table 2: Results of brightness measurements, performed with samples from Examples 1-3 and a Comparative Example. The values are given as relative values to the comparative sample, based on percentages.**

| | Comparative | Example 1 | Example 2 | Example. 3 |
|---|---|---|---|---|
| | zirconia: no additive | zirconia: with dysprosia | zirconia: with europia | zirconia: with samaria |
| brightness under daylight | 100 | 114.2 | 113.3 | 110.0 |
| brightness under daylight + UV light | 100 | 117,0 | 118.6 | 114.4 |
| brightness under neon light + UV light | 100 | 105,5 | 106.5 | 102.5 |

## Claims

1. A dental article comprising two sections, section A and section B,
section A having an upper and a lower surface portion, section A comprising
zirconia in an amount of at least about 80 mol% (with respect to section A),
a stabilizer and
a component X comprising Dy or Sm or Eu or mixtures thereof,
section B comprising a glass, glass ceramic or composite material,
section B being located above the upper surface portion of section A.

2. The dental article according to any of the preceding claims, wherein the stabilizer is selected from components comprising Y, Mg, Ca, Ce, La combinations and mixtures thereof.

3. The dental article according to any of the preceding claims, the stabilizer being present in an amount of about 1 to about 8 mol% with respect to section A.

4. The dental article according to any of the preceding claims, the amount of component X being in a range from about 0.1 to about 5 mol% with respect to section A (calculation based on the amount of oxide of the element referred to).

5. The dental article according to any of the preceding claims, the amount of stabilizer and component X being in a range of about 2 to about 10 mol% with respect to section A (calculation based on the amount of oxide of the element referred to).

6. The dental article according to any of the preceding claims, wherein section B meets at least one of the following parameters:
• thickness: about 0.01 to about 3 mm;
• opacity: about 20% to about 90% (determined on a 0.5 mm thick sample);
• melting range: about 500 to about 1000°C;
• bending strength: from about 50 to about 400 MPa (determined according to ISO 6872:2008).

7. The dental article according to any of the preceding claims having the shape of a dental crown, a dental bridge or abutment.

8. The dental article according to any of the preceding claims, wherein section A does not comprise components selected from Nd or Cu or Cr or Ti, or combinations or mixtures thereof in an amount above about 0.012 wt% with respect to the weight of section A.

9. The dental article according to any of the preceding claims, wherein the glass or glass ceramic material of section B is **characterized by** at least one of the following features:
• comprising about 55 wt% to about 75 wt% of silicon oxide, about 8 wt% to about 22 wt% of aluminum oxide, about 0 wt% to about 8 wt% of boron oxide, about 3 wt% to about 12 wt% of potassium oxide, about 4 wt% to about 12 wt% of sodium oxide, about 0 wt% to about 2 wt% of cerium oxide, about 0 wt% to about 2 wt% of tin oxide, about 0 wt% to about 3 wt% of zinc oxide, about 0 wt% to about 4 wt% of phosphor oxide, about 0 wt% to about 3 wt% of calcium oxide, about 0 wt% to about 3 wt% of lithium oxide, about 0 wt% to about 1 wt% of fluoride, about 0 wt% to about 3 wt.% of lanthanum oxide or lanthanide oxide;
• coefficient of thermal expansion: about 8 * 10⁻⁶K⁻¹ to about 15.8 * 10⁻⁶K⁻¹
• mean particle size: from about 1 µm to about 60 µm (measured with laser diffraction);
• melting temperature (range): below about 1000 °C;
• density: about 2.0 to about 2.8.

10. Process for producing a dental article as described in any of the preceding claims comprising the steps of
a) providing an article comprising
zirconia in an amount of at least about 85 mol%,
a stabilizer and
a component X comprising Dy or Sm or Eu or mixtures thereof,
b) covering at least a part of the surface of the article with a glass or glass ceramic material,
c) optionally firing the article the surface of which has at least been partially covered with a glass or glass ceramic material.

11. Process according to the preceding claim, wherein at least part of the surface of the article is treated with a composition comprising Eu before step b).

12. Process according to claim 10 or 11, wherein the article has the shape of a dental mill blank or a dental support structure.

13. Kit of parts comprising part 1 and part 2,
part 1 comprising a blank comprising zirconia in an amount of at least about 80 mol%, a stabilizer and a component X being selected from Dy or Sm or Eu or mixtures thereof,
part 2 comprising a glass, glass ceramic or composite material,
wherein the blank is to be used for the production of a dental article as described in any of claims 1 to 9.

14. The kit of parts according to the preceding claim, the blank being fixed to a holding device or frame.

15. Use of a blank as described in any of claims 13 or 14 for producing a dental article as described in any of claims 1 to 9.
